# EUROPÄISCHE PATENTSCHRIFT

(11) **EP 3 562 973 B1**
(45) Veröffentlichungstag und Bekanntmachung des Hinweises auf die Patenterteilung: **21.06.2023**
(21) Anmeldenummer: 18706203.9
(22) Anmeldetag: 01.02.2018
(51) Int. Cl.: C25B 15/02, C25B 3/25

(54) **GEPULSTE ELEKTROLYSE MIT BEZUG AUF DIE LEERLAUFSPANNUNG**
PULSED ELECTROLYSIS WITH REFERENCE TO THE OPEN CIRCUIT VOLTAGE
ELECTROLYSE PULSÉE PAR RAPPORT À LA TENSION EN CIRCUIT OUVERT

(30) Priorität: 08.02.2017 DE 102017201988
(43) Veröffentlichungstag der Anmeldung: 06.11.2019
(73) Patentinhaber: Siemens Energy Global GmbH & Co. KG, 81739 München (DE)
(72) Erfinder: ENGELBRECHT, Andreas, 95503 Hummeltal (DE); FLEISCHER, Maximilian, 85635 Höhenkirchen (DE); HÄMMERLE, Martin, 95447 Bayreuth (DE); MOOS, Ralf, 95447 Bayreuth (DE); WIESNER-FLEISCHER, Kerstin, 85635 Höhenkirchen-Siegertsbrunn (DE)
(86) Internationale Anmeldenummer: PCT/EP2018/052548
(87) Internationale Veröffentlichungsnummer: WO 2018/145996

(56) Entgegenhaltungen:
- WO-A1-02/077633
- WARNER T E ET AL: "A fundamental study of a novel dynamic electrochemical technique for the measurement of trace amounts of arsenic and antimony in molten zinc", SOLID STATE IO, NORTH HOLLAND PUB. COMPANY. AMSTERDAM; NL, NL, Bd. 136-137, 2. November 2000 (2000-11-02) , Seiten 589-601, XP004225990, ISSN: 0167-2738, DOI: 10.1016/S0167-2738(00)00342-8
- LEE J ET AL: "Electrocatalytic activity of Cu electrode in electroreduction of CO"2", ELECTROCHIMICA A, ELSEVIER SCIENCE PUBLISHERS, BARKING, GB, Bd. 46, Nr. 19, 15. Juni 2001 (2001-06-15) , Seiten 3015-3022, XP004247310, ISSN: 0013-4686, DOI: 10.1016/S0013-4686(01)00527-8

## Beschreibung

Die vorliegende Erfindung betrifft ein Elektrolyseverfahren, bei dem eine gepulste Spannung zwischen einer Anode und einer Kathode angelegt wird. Darüber hinaus betrifft die vorliegende Erfindung eine Elektrolysevorrichtung mit einer Anode, einer Kathode, einem Referenzsystem und einer Spannungsquelle zum Anlegen einer gepulsten Spannung zwischen der Anode und der Kathode.

In einer Elektrolysevorrichtung mit einem geeigneten Elektrolyt kann durch ein übliches Verfahren CO₂ oder CO als Edukt unter Zuhilfenahme eines üblicherweise kupferhaltigen Katalysators in einem einstufigen Prozess zu hochwertigeren Produkten wie CH₄, C₂H₄, C₂H₆ oder auch Alkoholen, Aldehyden oder Säuren umgesetzt werden. Dabei wird, wie auch bei der Wasserelektrolyse üblich, ein stationärer Betriebspunkt, d.h. eine konstante Stromdichte, oder auch ein konstantes Potential an der Arbeitselektrode angestrebt. An kupferhaltigen Katalysatoren werden jedoch stets eine mangelnde Langzeitstabilität des Katalysators sowie eine Abnahme der Selektivität für kohlenstoffhaltige Reduktionsprodukte wie z.B. CH₄, C₂H₄, beobachtet. Hierbei nimmt stets die Konkurrenzreaktion der Wasserstoffentwicklung (HER) entsprechend zu. Dieses Phänomen wird bisher auf morphologische Veränderungen der Katalysatoroberfläche und dem damit einhergehenden Verlust aktiver Kristallorientierungen sowie auf Vergiftungserscheinungen, z.B. die Deaktivierung durch Ablagerung unlöslicher Reduktionsprodukte auf der Katalysatoroberfläche, wie Kohlenstoff oder anderer Verunreinigungen aus eingesetzten Reagenzien, zurückgeführt (Shiratsuchi et al., J. Elektrochem. Soc., Vol. 140, No. 12, 1993; Jermann et al., Elektrochimica Acta, Vol. 39, No. 11/12, Seiten 1891 bis 1896, 1994).

In der bislang veröffentlichten Literatur finden sich nur wenige Arbeiten, welche die Problematik der Langzeitstabilität fokussieren. 1993 haben Shiratsuchi et al. (siehe oben) Elektrolysen mit gepulstem Potential (kathodische und anodische Anteile) durchgeführt und gezeigt, dass mit verschiedenen Verhältnissen von anodischer zu kathodischer Pulsdauer - es ergibt sich dabei ein rechteckförmiges Spannungsprofil - eine Steigerung der Faraday-Effizienz (FE) für CH₄ und C₂H₄ bis zu ca. 20 % möglich ist. Zudem wird dargelegt, dass sich bei einer Elektrolysedauer von ca. 25 Stunden die Faraday-Effizienz für Methan bei gepulstem Betrieb auf knapp über 10 % steigert, während sie bei konstantem Potential schon nach ca. 6 Stunden auf unter 1 % abgesunken ist. Die Effizienz für Ethen steigt auf etwa 25 %, scheint jedoch ab knapp 17 Stunden wieder abzusinken. Bemerkenswert ist auch die beobachtete Unterdrückung der H₂-Bildung (ca. 20 % FE gepulst gegenüber ca. 80 % FE in konstantem Betrieb).

Lee et al. (Lee et al., Electrochimica Acta, 46, Seiten 3015-3022, 2001) arbeiten ebenfalls mit einem Rechteck-Pulsbetrieb und weisen eine Zunahme der Elektrodenmasse bei konstantem Betrieb und eine Abnahme der Elektrodenmasse bei gepulstem Betrieb nach. Sie schlussfolgern daraus die Lösung von Kupferionen bzw. Bildung von Cu₂O über Kupferhydroxid als Intermediat. Die Faraday-Effizienzen betragen für CH₄ jedoch nur maximal 20 %, die für C₂H₄ ist kleiner als 5 %.

Eine breiter angelegte Parameterstudie wurde 1994 von Nogami et al. (Nogami et al., J. Elektrochem. Soc., Vol. 141, No. 5, 1994) durchgeführt. Dabei werden bei bestimmten Bedingungen Faraday-Effizienzen für Methan von 50 % über eine Elektrolysedauer von 10 Stunden erreicht. Die Effizienz für Ethen kommt jedoch nur auf knapp 10 %.

Ein weiterer Ansatz wurde von Jermann et al. (siehe oben) erprobt. Dabei wurden alle 5 Minuten drei sägezahnförmige Spannungspulse zu einem sehr hohen anodischen Potential auf 1,1 V gegenüber Ag/AgCl eingefügt, wobei der Fokus auf der Reaktivierung der Elektrode durch Entfernung deaktivierende Spezies auf der Oberfläche liegt. Die gezeigte Erhaltung der FE für CH₄ von >40 % über mehr als 45 Stunden hinweg konnte jedoch mit dieser Pulsmethode nicht reproduziert werden.

Als Fazit lässt sich feststellen, dass Potentialpulse bereits beschrieben wurden, um die Langzeitstabilität des Katalysators zu verbessern und die Produktselektivität zu steuern. Durch den Pulsbetrieb wird aber neben der Energie für die Erzeugung der Produkte auch Energie für die Regenerierung/Aktivierung des Katalysators benötigt. Letzteres ist ein Verlust, der eine wirtschaftliche Nutzung unrentabel machen kann.

Aus dem Artikel "Electrocatalytic activity of Cu electrode in electroreduction of CO2" von J. Lee, Y. Tak, Electrochim, Acta 2001,46,3015-3022 ist eine gepulste Elektroreduktion bekannt. Bei einem OCP von -0,05 V wird ein kathodisches Potential von -2,1 V für 10 s und anschließend ein anodisches Potential von 0,0 V für 5 s an eine Kupferelektrode gelegt.

Die Druckschrift DE 10 2013 105 605 A1 offenbart ein Verfahren zu elektrolytischen Synthese von Methanol und Methan. Für einen höheren Wirkungsgrad wird eine gepulste Stromversorgung eingesetzt. Das Betreiben bei umgepolter Stromversorgung kann regelmäßig oder bedarfsweise erfolgen.

Die Aufgabe der vorliegenden Erfindung besteht somit darin, ein Elektrolyseverfahren bereitzustellen, mit dem die Faraday-Effizienz über einen längeren Zeitraum hochgehalten werden kann. Darüber hinaus soll eine entsprechende Elektrolysevorrichtung angegeben werden.

Erfindungsgemäß wird diese Aufgabe gelöst durch ein Elektrolyseverfahren nach Anspruch 1 sowie eine Elektrolysevorrichtung nach Anspruch 13. Vorteilhafte Weiterbildungen der Erfindung ergeben sich aus den Unteransprüchen.

Entsprechend der vorliegenden Erfindung wird demnach ein Elektrolyseverfahren bereitgestellt, bei dem eine gepulste Spannung zwischen einer Anode und einer Kathode angelegt wird oder ein gepulster Strom eingeprägt wird. Die Elektrolysezelle wird also nicht mit Gleichspannung, sondern mit einer speziellen Wechselspannung, nämlich einer gepulsten Spannung oder mit einem speziellen Wechselstrom, nämlich einem gepulsten Strom betrieben. Vorzugsweise wird hierzu ein Potentiostat oder ein Galvanostat verwendet. Unter einer gepulsten Spannung wird hier nicht nur ein rechteckiges Spannungsprofil verstanden, sondern auch Profile, die Flanken mit schrittweiser oder kontinuierlicher Änderung des Potentials für die Übergänge zwischen kathodischem und anodischem Potential oder umgekehrt aufweisen. Gleiches gilt für den gepulsten Strom.

Es erfolgt bei dem Elektrolyseverfahren ein wiederholtes Messen eines jeweils aktuellen OCPs (open circuit potential, deutsch: Leerlaufspannung), welches die Kathode in einem unbestromten Zustand gegenüber einem Referenzsystem besitzt, oder alternativ ein Vorgeben eines OCP-Verlaufs. Es wird also dynamisch bzw. fortlaufend das aktuelle OCP jeweils als momentaner Einzelwert bestimmt, wobei die Anode und die Kathode elektrisch voneinander getrennt sind.

Im Rahmen des Elektrolyseverfahrens erfolgt nun ein Regeln der gepulsten Spannung oder des gepulsten Stroms zwischen der Anode und der Kathode derart, dass ein Arbeitspotential der Kathode im bestromten Zustand gegenüber dem Referenzsystem einen vorgegebenen Verlauf relativ zu dem jeweils aktuellen OCP besitzt. Es wird also das momentane beziehungsweise aktuelle OCP, d.h. das Kathodenpotential im unbestromten Zustand gegenüber dem Referenzsystemals Bezugsgröße für das Potential der Kathode im bestromten Zustand, d.h. während des Elektrolysebetriebs, verwendet. Dazu wird die Spannung oder der Strom zwischen der Anode und der Kathode mittels Regelung so eingestellt, dass das Kathodenpotential gegenüber dem Referenzsystem einen vorgegebenen Soll-Verlauf besitzt. Dabei weist der vorgegebene Verlauf mindestens einen Abschnitt mit kathodischem Niveau und mindestens einen Abschnitt mit anodischem Niveau auf. Während des kathodischen Abschnitts läuft die gewünschte Elektrolyse ab, wohingegen während des anodischen Abschnitts die Regeneration der Kathode erfolgt. Für die Regeneration genügt es, wenn das Potential der Kathode kurzfristig über dem OCP liegt.

Vorzugsweise wird eine galvanostatische Betriebsführung gewählt, die einen vorgegeben Stromverlauf mit einer Abfolge von Abschnitten mit anodischem und kathodischem Stromniveau aufweist, wobei der vorgegebene Stromverlauf dynamisch mit dem gemessenen OCP nachjustiert wird. Bei einer Erstmessung ist es sinnvoll, das OCP, auf das der Stromverlauf ausgerichtet werden soll, ständig aktuell zu ermitteln. Bei feststehenden Prozessen ist es unter Umständen günstiger, einen vorgegebenen OCP-Verlauf zu nutzen.

In einem Anwendungsbeispiel wird bei dem Elektrolyseverfahren in einem einstufigen Prozess aus Kohlenmonoxid oder Kohlendioxid ein Kohlenwasserstoff gewonnen. Mit der oben definierten spezifischen Pulsspannung relativ zu dem OCP lässt sich so der Kohlenwasserstoff mit einer hohen Faraday-Effizienz erzeugen. Mithin kann entsprechend viel Energie eingespart werden.

Vorzugsweise wird bei dem Elektrolyseverfahren ein Katalysator verwendet, der insbesondere kupferhaltig ist. In der anodischen Phase wird auf der Kathode Kupfer (I)-Oxid gebildet, welches die Bildung von C₂Hₓ katalysiert.

Bei dem Elektrolyseverfahren kann ein wässriger Elektrolyt verwendet werden. Dieser ist prozessfördernd und günstig in der Anschaffung.

Während des Analyseverfahrens kann sich das OCP ändern. Beispielsweise kann das OCP von einem Anfangswert auf einen systembedingten Sättigungswert absinken. In diesem Fall ist es günstig, wenn der Abschnitt des vorgegebenen Verlaufs mit anodischem Niveau auch entsprechend reduziert wird, sodass das "Umpolen" der Kathode etwas geringer ausfallen kann, wodurch sich Energie einsparen lässt.

Vorzugsweise wird als Referenzsystem ein Silber/Silberchlorid-System bzw. Ag/AgCl-System verwendet. Dabei stellt die Silberelektrode in der Silberchloridlösung die Bezugselektrode, beispielsweise für den Potentiostaten dar. Ein derartiges Referenzsystem ist äußerst zuverlässig, prinzipiell können aber auch andere Referenzelektroden beziehungsweise Referenzsysteme verwendet werden.

In einem Ausführungsbeispiel können das Arbeitspotential der Kathode im bestromten Zustand und das OCP der Kathode im unbestromten Zustand negativ sein, wobei gleichzeitig das Arbeitspotential der Kathode zeitweise auf anodischem Niveau liegt. Dies bedeutet, dass das anodische Niveau der Kathode auch negativ gewählt werden kann, wenn das OCP ausreichend negativ ist. Das anodische Potential muss also nicht zwangsläufig positiv sein, sondern kann tiefer liegen, wodurch wiederum Energie eingespart werden kann.

Insbesondere kann das Arbeitspotential der Kathode auf dem anodischen Niveau unterhalb eines Anfangs-OCPs liegen, das zu Beginn des Elektrolyseverfahrens messbar ist. Wie oben bereits angedeutet wurde, kann das OCP von einem Anfangs-OCP nach einer gewissen Betriebsphase auf einen Sättigungswert sinken. In diesem Fall ist es eben auch günstig, das Niveau des anodischen Abschnitts ebenfalls entsprechend abzusenken, damit die Umpolungsenergie reduziert wird.

In einem periodischen Betrieb kann regelmäßig wiederholt eine zeitliche Dauer des Abschnitts auf dem anodischen Niveau <10 s und eine zeitliche Dauer des Abschnitts auf dem kathodischen Niveau >10 s liegen. Vorzugsweise liegen die zeitliche Dauer des Abschnitts auf dem anodischen Niveau in einem Intervall von 2 s bis 7 s, insbesondere bei 5 s und die Dauer des Abschnitts auf dem kathodischen Niveau in einem Intervall von 20 s bis 100 s, insbesondere in einem Intervall von 25 s bis 50 s. Mit einer derartigen Pulsstruktur und der oben genannten Nachführung des anodischen Potentials kann eine Faraday-Effizienz von mehr als 50 % für die Bildung von C₂H₄, CH₄ und CO erreicht werden.

In einer Weiterbildung des erfindungsgemäßen Elektrolyseverfahrens wird eine Energie, die beim Umpolen der Kathode freigesetzt wird, in einem Zwischenspeicher gespeichert. Die zwischengespeicherte Energie lässt sich in einem Folgezyklus wieder nutzen. Durch diese Wiederverwendung lässt sich die Effizienz des Elektrolyseverfahrens steigern.

Erfindungsgemäß wird die oben genannte Aufgabe auch gelöst durch eine Elektrolysevorrichtung mit einer Anode, einer Kathode, einem Referenzsystem und einer Spannungsquelle zum Anlegen einer gepulsten Spannung oder einer Stromquelle zum Einprägen eines gepulsten Stroms zwischen der Anode und der Kathode, sowie mit einer Messeinrichtung zum wiederholten Messen eines jeweils aktuellen OCPs, welches die Kathode in einem unbestromten Zustand gegenüber dem Referenzsystem besitzt und einer Regelungseinrichtung zum Regeln der gepulsten Spannung oder des gepulsten Stroms zwischen der Anode und der Kathode derart, dass ein Arbeitspotential der Kathode im bestromten Zustand gegenüber dem Referenzsystem einen vorgegebenen Verlauf relativ zu dem jeweils aktuellen OCP besitzt, wobei der vorgegebene Verlauf mindestens einen Abschnitt mit kathodischem Niveau und mindestens einen Abschnitt mit anodischem Niveau aufweist.

Die oben im Zusammenhang mit dem erfindungsgemäßen Elektrolyseverfahren beschriebenen Variationsmöglichkeiten und Vorteile sind sinngemäß als funktionelle Merkmale auf die Elektrolysevorrichtung übertragbar.

Die vorliegende Erfindung wird nun anhand der beigefügten Zeichnungen näher erläutert, in denen zeigen:
- FIG 1: eine schematische Darstellung eines Pulsbetriebs mit Bezug auf das OCP-Potential;
- FIG 2: ein beispielhaftes Pulsprofil mit Spannungen gegen Ag/AgCl;
- FIG 3: die Faraday-Effizienz gasförmiger Produkte bei der Elektrolyse von CO₂;
- FIG 4: Teilstromdichten der gasförmigen Produkte;
- FIG 5: eine Abhängigkeit des anodischen Ladungsanteils vom anodischen Pulspotential;
- FIG 6: eine Stromantwort auf einen Pulszyklus;
- FIG 7: eine Faraday-Effizienz gasförmiger Produkte bei einem Pulsprogramm von 25 s, -1,6 V/5 s, +0,15 V;
- FIG 8: Teilstromdichten zu der Elektrolyse von FIG 7;
- FIG 9: die Faraday-Effizienz gasförmiger Produkte bei einem Pulsprogramm von 50 s, -1,6 V/5 s, +0,15 V und
- FIG 10: Teilstromdichten zu der Elektrolyse von FIG 9.

Die nachfolgend näher geschilderten Ausführungsbeispiele stellen bevorzugte Ausführungsformen der vorliegenden Erfindung dar. Dabei ist zu beachten, dass die einzelnen Merkmale nicht nur in den geschilderten Merkmalskombinationen, sondern auch in Alleinstellung oder in anderen technisch sinnvollen Kombinationen realisiert werden können.

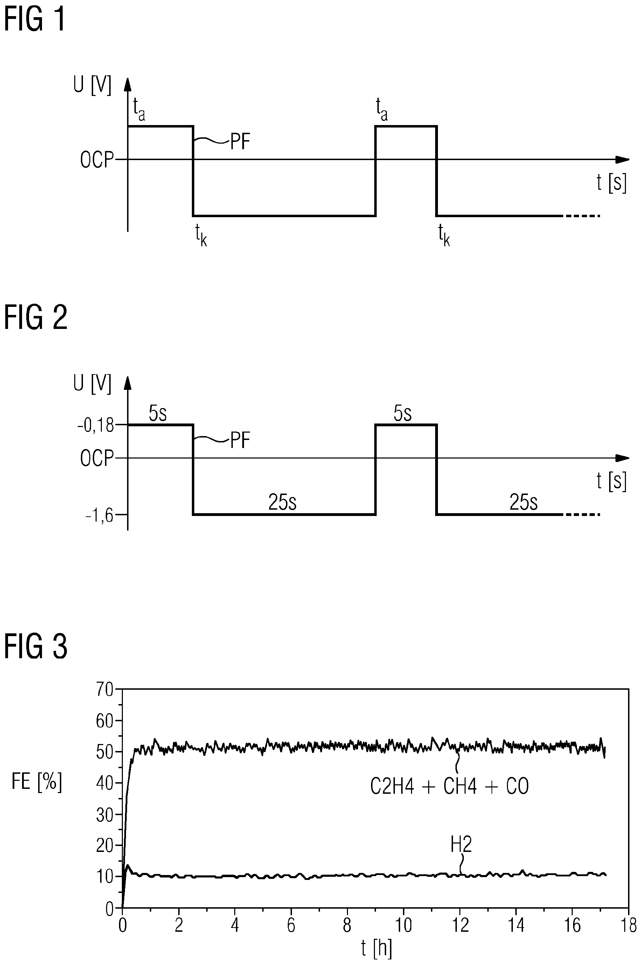

Statt des üblichen stationären Betriebspunkts (Potential/Stromdichte) wird beispielsweise bei der elektrochemischen Umsetzung von CO₂ oder CO zu Kohlenwasserstoffen eine gepulste Betriebsführung angewendet. Dabei wird, wie FIG 1 zeigt, die im Betrieb negativ polarisierte Arbeitselektrode (Kathode) periodisch in einen relativ zu dem OCP (open circuit potential, deutsch: Leerlaufspannung) des Systems anodischen Zustand umgepolt. Während das OCP das Potential der Kathode darstellt, wenn zwischen Kathode und Anode keine Spannung anliegt (unbestromter Zustand), zeigt die Kurve PF eine Pulsform bzw. ein Soll-Potential (vorgegebener Verlauf) der Kathode während des Elektrolysebetriebs (d.h. im bestromten Zustand). Dieses Soll-Potential besitzt anodische Abschnitte tₐ oberhalb des OCP und kathodischen Abschnitte tₖ unterhalb des OCP. Die Soll-Spannung beziehungsweise der vorgegebene Verlauf der Kathodenspannung gegenüber dem Referenzsystem (z.B. Ag/AgCl) ergibt sich beispielsweise, wenn mit einem Potentiostat zwischen Kathode und Anode eine entsprechend höhere Spannung angelegt wird. Der Potentiostat regelt dabei die Spannung zwischen Kathode und Anode so, dass sich zwischen der Kathode und der Referenzelektrode der in FIG 1 dargestellte Soll-Verlauf PF ergibt.

Bei der gepulsten Elektrolyse gilt es mehrere Aspekte zu betrachten. Zum einen ist von einem Reinigungseffekt an der Elektrodenoberfläche auszugehen. Dieser besteht allerdings nicht primär im Entfernen von Verunreinigungen, sondern vielmehr in der gezielten Korrosion der Katalysatoroberfläche (die nach einiger Zeit in konstantem Betrieb verminderte Aktivität aufweist) durch Bildung von Kupferhydroxiden/- carbonaten, respektive Kupfer (I)-Oxid und der anschließenden Reduktion zu Kupfer, welches sich wiederum unter Ausbildung von aktiven Hoch-Index-Flächen auf der Oberfläche abscheidet. Des Weiteren verbleibt auch gebildetes Kupfer (I)-Oxid auf der Oberfläche, welches anscheinend die Bildung von C₂Hₓ katalysiert.

Im Wesentlichen kann auch allgemein eine kontinuierliche (Wieder-) Bildung des Katalysators erreicht werden. Das Katalysatormaterial verbraucht sich und wird durch die Neubildung im Betrieb wiederhergestellt.

FIG 1 zeigt einen schematischen Pulsverlauf. Es gilt zu beachten, dass das OCP nicht zwangsläufig konstant ist. Vielmehr sinkt das OCP einer kupferhaltigen Kathode in einer wässrigen Lösung während des Elektrolysebetriebs beispielsweise ab. Bei der Elektrolysevorrichtung handelt es sich jedoch um ein Trägersystem, dessen OCP je nach Stärke der vorangegangenen Polarisation bis zu 1 Stunde brauchen kann, um wieder den ursprünglichen Wert zu erreichen, wenn er überhaupt erreicht wird.

Infolgedessen gibt es zwei Möglichkeiten, die Pulse so zu gestalten, dass die eingangs erwähnten Energieverluste möglichst gering werden:
a) Durch Reduzierung der Häufigkeit anodischer Pulse. Die anodischen Pulse werden nur zur Regeneration der katalytischen Eigenschaften der Kathode benötigt.
b) Die beschriebene Trägheit des Systems erlaubt es, mit Potentialen unterhalb des anfänglichen OCP einen anodischen Effekt zu erzielen, wenn das angelegte Potential anodisch zum momentanen OCP ist. Dies ist beispielsweise der Fall, wenn das OCP von anfänglich -0,12 V gegenüber Ag/AgCl auf einen momentanen Wert von -0,20 V gegenüber Ag/AgCl abfällt und das aktuelle anodische Niveau mit -0,18 V gegenüber Ag/AgCl gewählt wird. Im momentanen anodischen Betrieb liegt das Potential der Kathode (-0,18 V) unterhalb des anfänglichen OCP von -0,12 V aber oberhalb des aktuellen OCP von -0,20 V.

Eine Möglichkeit zur Energierückgewinnung ist durch das Zwischenspeichern der Energie beim Umpolen der Elektroden gegeben. Wenn der Regenerationsprozess-Zyklus durch ist, wird nicht kurzgeschlossen und die Energie vernichtet, sondern die nach dem Ende der Regeneration an den Elektroden vorliegende Spannung wird in einen Energiezwischenspeicher entladen und die Ladung wird beim kommenden Zyklus wiederverwendet.

Eine reine Unterbrechung des kathodischen Betriebs, d.h. kein anodischer Betrieb, führt hingegen nicht zu einer Verbesserung des Prozesses.

Im Zusammenhang mit den FIG 2 bis 9 werden nun einige Beispiele dargestellt, bei denen gepulste Elektrolyse mit Bezug auf das OCP-Potential durchgeführt wird.

In einem Beispiel wird das in FIG 2 dargestellte Pulsprofil verwendet. Das Pulsprofil zeigt den Soll-Verlauf der Spannung der Kathode während des Elektrolysebetriebs (bestromter Zustand der Kathode) gegenüber einem Referenzsystem Ag/AgCl. Es handelt sich um einen periodischen Pulsverlauf. Einem kurzen anodischen Abschnitt von 5 s folgt ein kathodischer Abschnitt mit 25 s. Dem schließt sich wieder ein anodischer Abschnitt mit 5 s an usw. Das Potential im anodischen Abschnitt liegt im vorliegenden Beispiel bei -0,18 V und das Potential im kathodischen Abschnitt bei -1,6 V. Das momentane OCP, das sich nach einer gewissen Betriebszeit eingestellt hat, liegt hier beispielsweise bei -0,20 V. Es liegt also deutlich unterhalb des ursprünglichen OCP von -0,12 V. Damit liegt aber auch der anodische Abschnitt unterhalb des ursprünglichen OCP aber oberhalb des momentanen OCP.

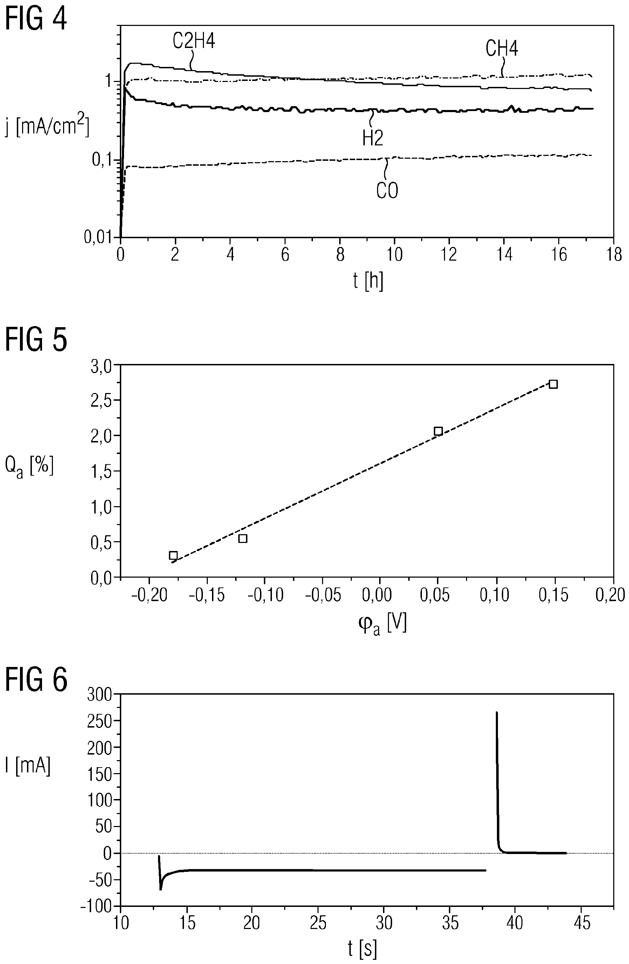

Mit der beschriebenen Betriebsweise ist es möglich, die H₂-Entwicklung, die als Konkurrenzreaktion zu den gewünschten Prozessen verstanden werden muss, zu unterdrücken. Bei geeigneter Wahl des Pulsprogramms (vergleiche FIG 2) gelang es, über 17 Stunden mit nur 10 % Faraday-Effizienz H₂ zu bilden, während CH₄, C₂H₄ und CO in der Summe mit einer konstanten Effizienz von 50 % gebildet wurden, was in FIG 3 dargestellt ist. Die Auswertung der Stromdichten j zeigt gemäß FIG 4 eine konstante Teilstromdichte für H₂, die von CO und CH₄ steigt leicht an und die Teilstromdichte von C₂H₄ nimmt ein wenig ab.

Im Zusammenhang mit FIG 5 wird nun der Effekt der Höhe des anodischen Potentials auf den Energieverbrauch verdeutlicht.

Es werden anodische Potentiale ϕₐ zwischen -0,18 V und +0,15 V untersucht. Bei einem anodischen Potential von +0,15 V gegenüber Ag/AgCl beträgt der Anteil Qₐ der anodischen Ladung, d.h. der Ladung, welche während des anodischen Pulses verbraucht wird, 2,7 % der insgesamt pro Pulszyklus eingesetzten Ladung. Bei einem anodischen Potential ϕₐ von -0,18 V gegenüber Ag/AgCl sind es nur noch 0,3 %.

Die Darstellung des Stromverlaufs I über der Zeit t für einen Pulszyklus in FIG 6 veranschaulicht das Ladungsverhältnis. Es wird ein exemplarischer Pulszyklus mit -1,6 V über 25 s und +0,15 V über 5 s verwendet. Während der kathodischen 25 s fließt etwa ein Strom von -30 mA. Während der anodischen 5 s fließt hingegen nahezu kein Strom. Beim Umpolen vom kathodischen in den anodischen Betrieb ist jedoch eine markante Stromspitze zu erkennen, die einen nicht zu vernachlässigen Energieverbrauch zur Folge hat. Dieser Energieverbrauch kann unter Umständen durch eine Zwischenspeicherung mittels eines Kondensators gemildert werden.

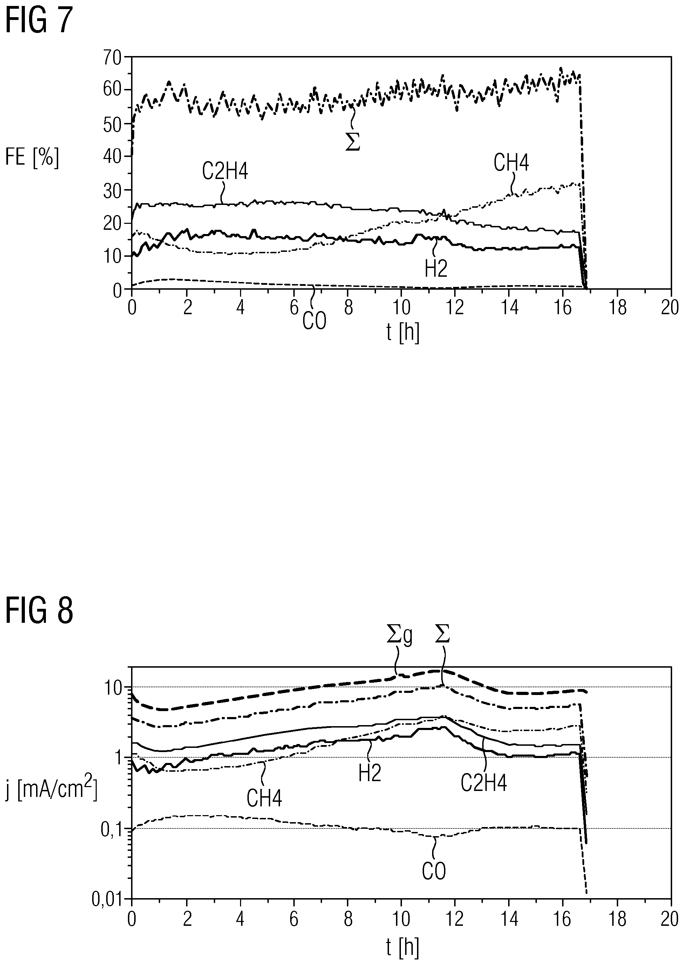

Mit einem Pulsprogramm, das in der Form demjenigen von FIG 2 entspricht, jedoch in den anodischen Anteilen bis zu einem Potential von +0,15 V gegenüber Ag/AgCl pulst, gelang es, die Selektivität für C₂H₄ über 8 Stunden gegenüber herkömmlichen Pulsformen zu erhöhen. Ein entsprechendes Resultat ist in FIG 7 dargestellt. In den ersten 10 Stunden liegt die Faraday-Effizienz für C₂H₄ über 25 %. Anschließend sinkt sie etwas ab und die Selektivität für CH₄ steigt an. Die Summe ∑ der Faraday-Effizienzen aller gasförmigen Produkte CO, H₂, CH₄ und C₂H₄ liegt über 50 %.

FIG 8 gibt die entsprechenden Teilstromdichten j wieder. Auch hier liegt die Teilstromdichte für C₂H₄ für einen Zeitraum von 10 Stunden deutlich über derjenigen von CH₄. Neben der Summe ∑ für alle gasförmigen Produkte ist auch die Gesamtsumme ∑_{g} für alle Produkte eingezeichnet, einschließlich der flüssigen Produkte.

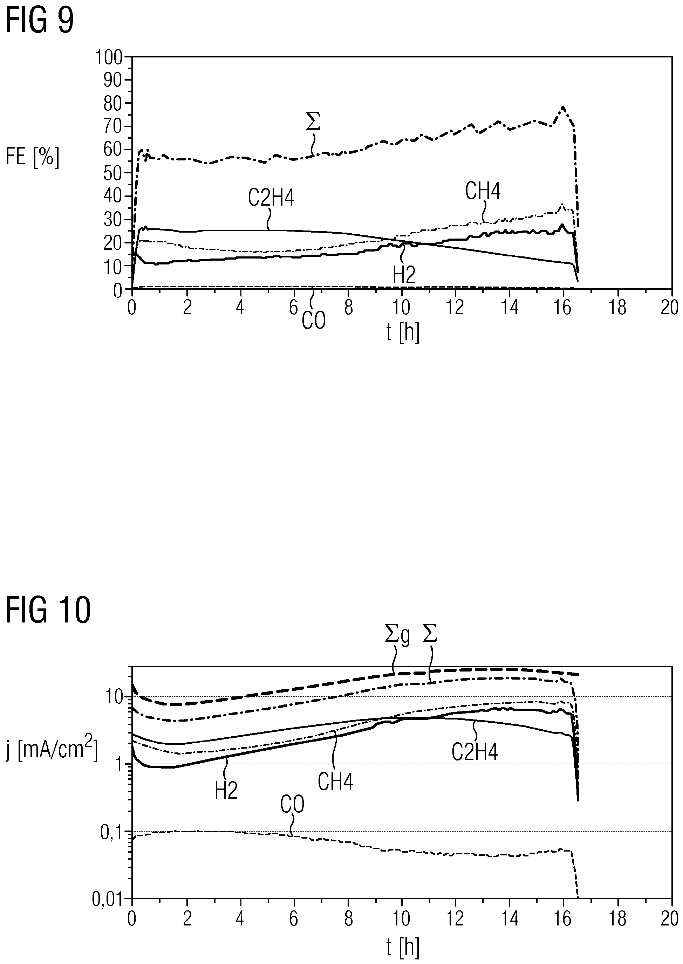

Weiterhin ist es möglich, die Anzahl der anodischen Pulse zu reduzieren, ohne den positiven Effekt auf die Produktbildung zu verlieren. Das Pulsprofil mit der längsten bisher erfolgreich überprüften kathodischen Reaktionszeit besteht aus einem kathodischen Abschnitt (-1,6 V) mit einer Dauer von 50 s, gefolgt von einem anodischen Puls (+0,15 V) von 5 s. Die resultierenden Faraday-Effizienzen sind in FIG 9 und die resultierenden Teilstromdichten in FIG 10 dargestellt. Auch hier ist zu erkennen, dass die Selektivität von C₂H₄ zumindest 8 Stunden über derjenigen von CH₄ liegt. Konkret liegt die Faraday-Effizienz für C₂H₄ über 8 Stunden etwa konstant bei 25 %.

Zusammenfassend lässt sich feststellen, dass entsprechend der vorliegenden Erfindung eine Pulsung in der Nähe des aktuellen OCP stattfindet, wodurch hohe Verluste bei Ladung und Energie vermieden werden.

Das OCP erholt sich nach kathodischer Polarisation (z.B. -1,6 V) nur sehr träge, sodass sogar mit Potentialen, die unterhalb des anfänglichen OCP liegen, aber anodisch zum momentanen OCP sind, eine anodische Polarisation und damit ein positiver Effekt, z.B. auf die Langzeitstabilität, erreicht werden kann.

Die Untersuchung verschiedener Pulsprogramme hat zudem gezeigt, dass es nicht sinnvoll ist, die Dauer des gesamten Pulszyklus zu stark zu verkürzen, da bei zu hoher Frequenz der Anteil kapazitiver Ströme und damit der Verluste steigt und der Prozess nicht mehr wirtschaftlich betrieben werden kann. Somit wäre es ebenso vorteilhaft, den kathodischen Anteil bei der Gestaltung der Pulsfolge, der für die Produktbildung relevant ist, möglichst lang auszubilden und den anodischen Anteil, der für die Langzeitstabilität und die Produktselektivität erforderlich ist, möglichst kurz zu halten. So hat z.B. eine Pulsfolge von 50 s kathodisch und 5 s anodisch einen positiven Effekt auf die Langzeitstabilität und die Produktselektivität gezeigt.

Das Vermeiden hoher Verluste durch Pulsung in der Nähe des OCP macht den Einsatz dieser Technik auch für größere Anlagen interessant. Dadurch würden stabile Langzeitelektrolysen in wässrigen Elektrolyten mit einer dauerhaften Unterdrückung der H₂-Entwicklung ermöglicht. Zudem ist es möglich, gleichzeitig die Selektivität/Bildungsrate werthaltiger Produkte wie C₂H₄ und CH₄ im Wesentlichen konstant zu halten und zwar unter Aufwendung geringster Energieanteile.

## Patentansprüche

1. Elektrolyseverfahren, umfassend die Schritte:
- Anlegen einer gepulsten Spannung oder Einprägen eines gepulsten Stroms zwischen eine Anode und eine Kathode, **gekennzeichnet durch**
- wiederholtes Messen eines jeweils aktuellen OCP, welches die Kathode in einem unbestromten Zustand gegenüber einem Referenzsystem besitzt, und
- Regeln der gepulsten Spannung oder des gepulsten Stroms zwischen der Anode und der Kathode derart, dass ein Arbeitspotential der Kathode im bestromten Zustand gegenüber dem Referenzsystem einen vorgegebenen Verlauf (PF) relativ zu dem jeweils aktuellen OCP besitzt, wobei
- der vorgegebene Verlauf (PF) mindestens einen Abschnitt (tₖ) mit kathodischem Niveau und mindestens einen Abschnitt (tₐ) mit anodischem Niveau aufweist.

2. Elektrolyseverfahren nach Anspruch 1, bei dem eine galvanostatische Betriebsführung gewählt wird, die einen vorgegeben Stromverlauf mit einer Abfolge von Abschnitten mit anodischem und kathodischem Stromniveau aufweist, wobei der vorgegebene Stromverlauf dynamisch mit dem gemessenen OCP nachjustiert wird.

3. Elektrolyseverfahren nach Anspruch 1 oder 2, bei dem in einem einstufigen Prozess aus Kohlenmonoxid oder Kohlendioxid ein Kohlenwasserstoff gewonnen wird.

4. Elektrolyseverfahren nach einem der vorhergehenden Ansprüche, bei dem ein insbesondere kupferhaltiger Katalysator verwendet wird.

5. Elektrolyseverfahren nach einem der vorhergehenden Ansprüche, bei dem ein wässriger Elektrolyt verwendet wird.

6. Elektrolyseverfahren nach einem der vorhergehenden Ansprüche, wobei sich das OCP während des Elektrolyseverfahrens ändert.

7. Elektrolyseverfahren nach einem der vorhergehenden Ansprüche, wobei als das Referenzsystem ein Silber/Silberchlorid-System verwendet wird.

8. Elektrolyseverfahren nach einem der vorhergehenden Ansprüche, wobei das Arbeitspotential der Kathode im bestromten Zustand und das OCP negativ sind und gleichzeitig das Arbeitspotential der Kathode zeitweise auf anodischem Niveau liegt.

9. Elektrolyseverfahren nach einem der vorhergehenden Ansprüche, wobei das Arbeitspotential der Kathode auf dem anodischen Niveau unterhalb eines Anfangs-OCPs, das zu Beginn des Elektrolyseverfahrens messbar ist, liegt.

10. Elektrolyseverfahren nach einem der vorhergehenden Ansprüche, wobei regelmäßig wiederholt eine zeitliche Dauer des Abschnitts (tₐ) auf dem anodischen Niveau unter 10s und eine zeitliche Dauer des Abschnitts (tₖ) auf dem kathodischen Niveau über 10s liegen.

11. Elektrolyseverfahren nach Anspruch 10, wobei die zeitliche Dauer des Abschnitts (tₐ) auf dem anodischen Niveau in einem Intervall von 2s bis 7s, insbesondere bei 5s, und die Dauer des Abschnitts (tₖ) auf dem kathodischen Niveau in einem Intervall von 20s bis 100s, insbesondere in einem Intervall von 25s bis 50s liegen.

12. Elektrolyseverfahren nach einem der vorhergehenden Ansprüche, wobei die gepulste Spannung oder der gepulste Strom ein rechteckförmiges, ein rampenförmiges oder ein mehrstufiges Profil aufweist.

13. Elektrolyseverfahren nach einem der vorhergehenden Ansprüche, wobei eine Energie, die beim Umpolen der Kathode freigesetzt wird, in einem Zwischenspeicher gespeichert wird.

14. Elektrolysevorrichtung mit
- einer Anode,
- einer Kathode,
- einem Referenzsystem und
- einer Spannungsquelle zum Anlegen einer gepulsten Spannung oder einer Stromquelle zum Einprägen eines gepulsten Stroms zwischen der Anode und der Kathode, **gekennzeichnet durch**
- eine Messeinrichtung zum wiederholten Messen eines jeweils aktuellen OCPs, welches die Kathode in einem unbestromten Zustand gegenüber dem Referenzsystem besitzt, und
- einer Regelungseinrichtung zum Regeln der gepulsten Spannung oder des gepulsten Stroms zwischen der Anode und der Kathode derart, dass ein Arbeitspotential der Kathode im bestromten Zustand gegenüber dem Referenzsystem einen vorgegebenen Verlauf (PF) relativ zu dem jeweils aktuellen OCP besitzt, wobei
- der vorgegebene Verlauf (PF) mindestens einen Abschnitt (tₖ) mit kathodischem Niveau und mindestens einen Abschnitt (tₐ) mit anodischem Niveau aufweist.

## Claims

1. Electrolysis method comprising the steps of:
- applying a pulsed voltage or establishing a pulsed current between an anode and a cathode,
**characterized by**
- repeated measurement of a respective current OCP that the cathode possesses in a zero-current state with respect to a reference system, and
- controlling the pulsed voltage or the pulsed current between the anode and the cathode such that a working potential of the cathode in the current-carrying state with respect to the reference system has a defined progression (PF) relative to the respective current OCP, wherein
- the defined progression (PF) has at least one phase (tₖ) at a cathodic level and at least one phase (tₐ) at an anodic level.

2. Electrolysis method according to Claim 1, in which a galvanostatic operating regime having a defined current progression with a sequence of phases with anodic and cathodic current level is chosen, wherein the defined current progression is readjusted dynamically with the measured OCP.

3. Electrolysis method according to Claim 1 or 2, in which a hydrocarbon is obtained in a one-stage process from carbon monoxide or carbon dioxide.

4. Electrolysis method according to any of the preceding claims, in which a catalyst, especially a copper-containing catalyst, is used.

5. Electrolysis method according to any of the preceding claims, in which an aqueous electrolyte is used.

6. Electrolysis method according to any of the preceding claims, wherein the OCP changes during the electrolysis method.

7. Electrolysis method according to any of the preceding claims, wherein the reference system used is a silver/silver chloride system.

8. Electrolysis method according to any of the preceding claims, wherein the working potential of the cathode in the current-carrying state and the OCP are negative and, at the same time, the working potential of the cathode is temporarily at an anodic level.

9. Electrolysis method according to any of the preceding claims, wherein the working potential of the cathode at the anodic level is below an initial OCP measurable at the start of the electrolysis method.

10. Electrolysis method according to any of the preceding claims, wherein there is regular repetition of a duration of the phase (tₐ) at the anodic level below 10 s and a duration of the phase (tₖ) at the cathodic level above 10 s.

11. Electrolysis method according to Claim 10, wherein the duration of the phase (tₐ) at the anodic level is within a range from 2 s to 7 s, especially 5 s, and the duration of the phase (tₖ) at the cathodic level is within a range from 20 s to 100 s, especially within a range from 25 s to 50 s.

12. Electrolysis method according to any of the preceding claims, wherein the pulsed voltage or the pulsed current has a square wave profile, a stepped profile or a multilevel profile.

13. Electrolysis method according to any of the preceding claims, wherein any energy which is released on reversal of polarity of the cathode is stored in an intermediate storage means.

14. Electrolysis apparatus having
- an anode,
- a cathode,
- a reference system and
- a voltage source for applying a pulsed voltage or a current source for establishing a pulsed current between the anode and the cathode,
**characterized by**
- a measurement unit for repeated measurement of a respective current OCP that the cathode possesses in a zero-current state with respect to the reference system, and
- a control unit for controlling the pulsed voltage or the pulsed current between the anode and the cathode such that a working potential of the cathode in the current-carrying state with respect to the reference system has a defined progression (PF) relative to the respective current OCP, wherein
- the defined progression (PF) has at least one phase (tₖ) at a cathodic level and at least one phase (tₐ) at an anodic level.

## Revendications

1. Procédé d'électrolyse, comprenant les stades :
- l'application d'une tension pulsée ou l'application d'un courant pulsé entre une anode et une cathode,
**caractérisé par**
- la mesure répétée d'une OCP respectivement en cours, que la cathode possède, dans un état non alimenté en courant par rapport à un système de référence, et
- la régulation de la tension pulsée ou du courant pulsé entre l'anode et la cathode, de manière à ce que un potentiel de travail de la cathode, dans l'état alimenté en courant par rapport au système de référence, possède une courbe (PF) donnée à l'avance par rapport à l'OCP respective en cours, dans lequel
- la courbe (PF) donnée à l'avance a au moins une partie (tₖ) ayant un niveau cathodique et au moins une partie (tₐ) ayant un niveau anodique.

2. Procédé d'électrolyse suivant la revendication 1, dans lequel on choisit une conduite de fonctionnement galvanostatique, qui a une courbe de courant donnée à l'avance ayant une succession de parties ayant un niveau de courant anodique et cathodique, dans lequel on réajuste la courbe de courant donnée à l'avance dynamiquement avec l'OCP mesurée.

3. Procédé d'électrolyse suivant la revendication 1 ou 2, dans lequel on obtient, dans un processus à un stade, un hydrocarbure à partir de monoxyde de carbone ou de dioxyde de carbone.

4. Procédé d'électrolyse suivant l'une des revendications précédentes, dans lequel on utilise un catalyseur, en particulier contenant du cuivre.

5. Procédé d'électrolyse suivant l'une des revendications précédentes, dans lequel on utilise un électrolyte aqueux.

6. Procédé d'électrolyse suivant l'une des revendications précédentes, dans lequel l'OCP se modifie pendant le procédé d'électrolyse.

7. Procédé d'électrolyse suivant l'une des revendications précédentes, dans lequel on utilise un système argent / chlorure d'argent comme système de référence.

8. Procédé d'électrolyse suivant l'une des revendications précédentes, dans lequel le potentiel de travail de la cathode, dans l'état non alimenté en courant, et l'OCP sont négatifs, et en même temps le potentiel de travail de la cathode est de temps en temps au niveau anodique.

9. Procédé d'électrolyse suivant l'une des revendications précédentes, dans lequel le potentiel de travail de la cathode est au niveau anodique en-dessous d'un OCP de début, que l'on peut mesurer au début du procédé d'électrolyse.

10. Procédé d'électrolyse suivant l'une des revendications précédentes, dans lequel, d'une manière répétée régulièrement, une durée dans le temps de la partie (tₐ) au niveau anodique est inférieure à 10 s et une durée dans le temps de la partie (tₖ) au niveau cathodique est supérieure à 10 s.

11. Procédé d'électrolyse suivant la revendication 10, dans lequel la durée dans le temps de la partie (tₐ) au niveau anodique est dans un intervalle de 2 s à 7 s, en étant en particulier de 5 s, et la durée de la partie (tₖ) au niveau cathodique est dans un intervalle de 20 s à 100 s, en étant en particulier dans un intervalle de 25 s à 50 s.

12. Procédé d'électrolyse suivant l'une des revendications précédentes, dans lequel la tension pulsée ou le courant pulsé a une forme rectangulaire, une forme en rampe ou un profil à plusieurs paliers.

13. Procédé d'électrolyse suivant l'une des revendications précédentes, dans lequel on accumule, dans un accumulateur intermédiaire, une énergie, que l'on libère lors du changement de polarité de la cathode.

14. Installation d'électrolyse comprenant
- une anode,
- une cathode,
- un système de référence, et
- une source de tension pour l'application d'une tension pulsée ou une source de courant pour l'application d'un courant pulsé entre l'anode et la cathode,
**caractérisée par**
- un dispositif de mesure pour la mesure répétée d'une OCP respectivement en cours que possède la cathode, dans un état non alimenté en courant par rapport au système de référence, et
- un dispositif de régulation pour réguler la tension pulsée ou de le courant pulsé entre l'anode et la cathode, de manière à ce qu'un potentiel de travail de la cathode, dans l'état alimenté en courant par rapport au système de référence, possède une courbe (PF) donnée à l'avance par rapport à l'OCP respective en cours, dans lequel
- la courbe (PF) donnée à l'avance a au moins une partie (tₖ) ayant un niveau cathodique et au moins une partie (tₐ) ayant un niveau anodique.
